# EUROPEAN PATENT APPLICATION

(11) **EP 1 836 949 A2**
(43) Date of publication of application: **26.09.2007**
(21) Application number: 07251213.0
(22) Date of filing: 22.03.2007
(51) Int. Cl.: A61B 1/04, A61B 1/005, A61B 1/12

(54) **Disposable endoscope devices**

(30) Priority: 23.03.2006 US 277290
(71) Applicant: ETHICON ENDO-SURGERY, INC., Cincinnati, Ohio 45242 (US)
(72) Inventor: Ortiz, Mark S., Milford, Ohio 45150 (US); Plescia, David, Cincinnati, Ohio 45227 (US)
(74) Representative: Tunstall, Christopher Stephen

(57) **Abstract**

Various methods and devices are provided for endoscopic procedures. In particular, the device can include an elongate flexible insertion element adapted for delivery within a patient. In one embodiment, the insertion element has at least one working channel disposed therein which is adapted to receive a surgical instrument, a liquid, or a gas. The insertion element can be adapted, for example, for endoscopic or laparoscopic delivery to a patient. The device further includes an optics unit disposed on a distal end of the insertion element that can be adapted to acquire images during endoscopic procedures, and an image display screen, disposed on or remote from the endoscopic device, adapted to communicate with the optics unit to display the acquired images.

## Description

### FIELD OF THE INVENTION

The invention relates to surgical devices, particularly endoscopic devices with optical capabilities.

### BACKGROUND OF THE INVENTION

Minimally invasive surgical procedures are widely used since a smaller incision tends to reduce the post-operative recovery time and complications. Moreover, advances in technology has made more surgical procedures amenable to minimally invasive techniques. Endoscopic and laparoscopic surgical devices, in particular, have become more commonly used in a wide range of procedures. Such devices typically are able to access a surgical site through a natural body orifice or through a small incision. Among the functionalities required of such devices is the ability to enable the surgeon to see the surgical site, which is often remote and not visible to the naked eye. While endoscopes that have or are able to adapted for use with optical systems are known, such systems can be bulky and tend to be quite costly. In addition, the image display systems available are further costly. Such optical systems can also be difficult to clean and reuse after a procedure.

Accordingly, there is a need for improved, economical endoscopic devices, particularly devices that offer enhanced versatility and that can be entirely or partially disposed of after use.

### SUMMARY OF THE INVENTION

The present invention generally provides devices and methods for medical treatment using a medical treatment device. While this can be achieved using a variety of techniques and devices, in one embodiment, the device can include an elongate flexible insertion element adapted for delivery within a patient. In one embodiment, the insertion element has at least one working channel disposed therein which is adapted to receive a surgical instrument, a liquid, or a gas. The insertion element can be adapted, for example, for endoscopic or laparoscopic delivery to a patient. The device further includes an optical image gathering unit disposed on a distal portion of the insertion element that can be adapted to acquire images during medical treatment procedures, and an image display screen disposed on a proximal portion of the medical treatment device adapted to communicate with the optical image gathering unit to display the acquired images. In one embodiment, the medical treatment device can be an endoscope.

In one exemplary embodiment, the optical image gathering unit can include a lens disposed on a distal end thereof adapted to focus images, and an LED disposed on the distal end thereof adapted to illuminate the area surrounding the optical image gathering unit. The optical image gathering unit can further include a lens cleaning sprayer disposed on the distal end thereof adapted to clean the lens. The lens cleaning sprayer can be coupled to a fluid inlet port disposed on the proximal end of the device through a fluid delivery device adapted to delivery lens cleansing fluid to the lens cleanser. In one embodiment, the optical image gathering unit can include piezoelectric material disposed under the lens which is adapted to move the lens to facilitate zoom.

The optical image gathering unit can be disposed on at least a portion of a flexible tip located on the distal end of the insertion element. The insertion element can include one or more cables disposed therein and attached to a control member disposed on the proximal portion of the device. The cables can be adapted to effect bending of a distal portion of the insertion element in response to actuation of the control member.

In one exemplary embodiment, a digital imaging chip can be disposed on the distal portion of the flexible tip to collect images from the lens. The digital imaging chip can be in electrical communication with the image display screen to allow the image display screen to display images acquired by the optics unit, and can communicate with the image display screen using, for example, wireless RF technology. The digital imaging chip can be connected to a processing board disposed in the proximal portion of the device through one or more wires adapted to process the images and transmit the images to the image display screen. In one embodiment, the processing board is adapted to stream the images acquired by the optical image gathering unit to an external display such that the images can be viewed both on the image display screen and the external display. The images can be streamed to the external display using a variety of formats, including USB and NTSC.

In another exemplary embodiment of an optics module for use with a medical treatment device, the module can include a lens disposed on a distal end of an optics unit adapted to acquire images. The optics module can be disposable and can be adapted to be removably and replaceably mated to a distal portion of a medical treatment device such that it is able to communicate with an image display screen to display images acquired by the optics module.
The image display screen can be disposed on a proximal portion of the medical treatment device, and can be removable mated to the proximal portion of the medical treatment device or can be fixedly disposed on the proximal portion of the medical treatment device. The optics unit can include piezoelectric material disposed under the lens, the piezoelectric material adapted to move the lens to facilitate zoom. In one embodiment, the optics module can further includes an LED disposed on the distal end of the optics unit adapted to illuminate the area surrounding the optics unit.

The medical treatment device can be, for example, an endoscope or can be adapted for laparoscopic delivery. The medical treatment device can include a processor that is adapted to stream the images acquired by the optics module to an external display such that the images can be viewed both on the image display screen and the external display. The images can be streamed to the external display using a variety of formats, including USB and NTSC.

### BRIEF DESCRIPTION OF THE DRAWINGS

The invention will be more fully understood from the following detailed description taken in conjunction with the accompanying drawings, in which:

FIG. 1 is an illustration of a disposable endoscopic device and an optics unit for acquiring images during an endoscopic procedure;

FIG. 2 is an illustration of the proximal end of the endoscopic device shown in FIG. 1;

FIG. 3 is an illustration of the distal end of the endoscopic device shown in FIG. 1;

FIG. 4A is an illustration of an embodiment of the optics unit shown in FIG. 1,

FIG. 4B is an illustration of another embodiment of the optics unit shown in FIG. 1

FIG. 5 is an illustration of an embodiment of an endoscopic device having a removable optics unit;

FIG. 6. is an illustration of the distal end of the endoscopic device shown in FIG. 5; and

FIG. 7 is an illustration of the proximal end of the endoscopic device shown in FIG. 5.

### DETAILED DESCRIPTION OF THE INVENTION

Certain exemplary embodiments will now be described to provide an overall understanding of the principles, structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for medical treatment using an endoscopic device. In particular, the methods and devices are configured to provide fully disposable endoscopic devices with optical capabilities or, alternatively, endoscopic devices with disposable optical components.

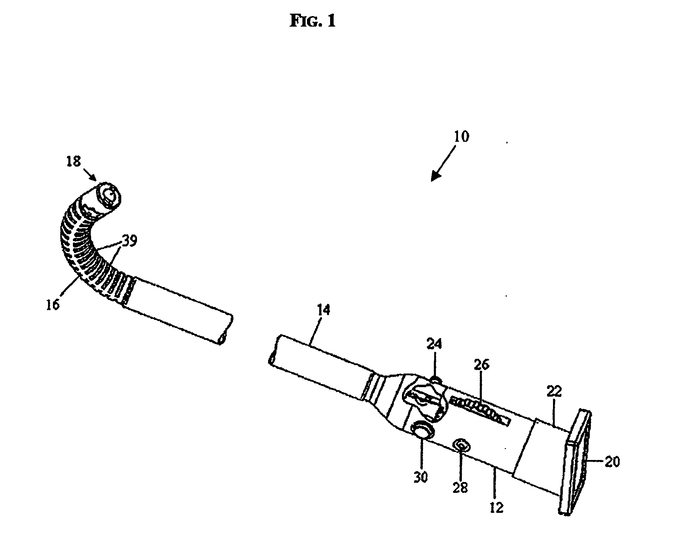

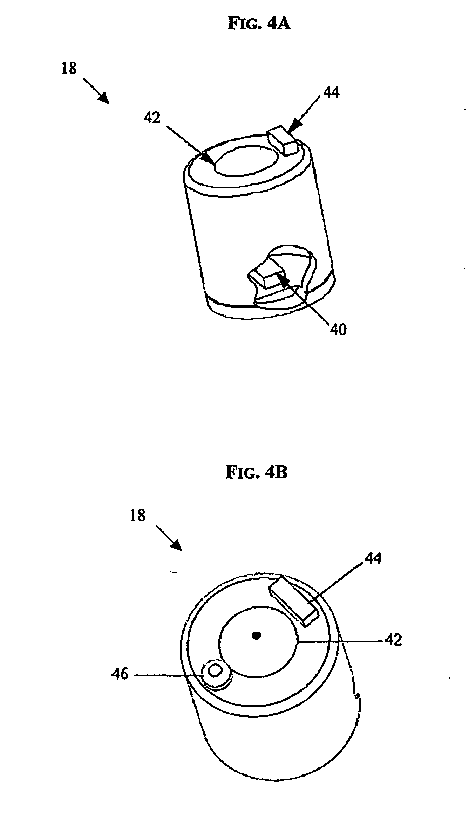

In one aspect, there is provided an endoscopic surgical treatment device and system, which includes on-board optics, that is entirely disposable. While various endoscopic devices can be used, FIG. 1 illustrates one exemplary embodiment of a disposable endoscopic device 10 that includes image gathering and display functionality. As shown, the endoscopic device 10 includes a handle portion 12, disposed on the proximal end of the device 10 that is adapted to allow a user to grip the endoscopic device 10, and an elongate flexible shaft 14 extending from the handle 12. A flexible and bendable tip 16 is disposed on the distal end of the shaft 14 and is adapted to flex and bend to allow the device to navigate through a curved pathway within a patient and/or to enable selective movement of an optical image gathering unit 18. The optical image gathering unit 18, shown in FIGS. 4A-4B, can be disposed on the distal end of the flexible tip 16 and is adapted to acquire images during an endoscopic procedure. The optical image gathering unit 18 is in communication with an LCD display screen 20, which can be disposed on a portion of the handle 12 and which is adapted to display the acquired images to a user of the endoscopic device 10.

The handle 12 can have any shape and size to facilitate gripping of the device. Moreover, the handle 12 can have a variety of control elements that can be manipulated by a user to control the endoscopic device and its functions, as explained below. The shaft 14 extending from the distal end of the handle 12 can include one or more inner channels (not shown) for receiving surgical instruments, liquids for irrigation or suction, gases, or any other materials for use in an endoscopic surgery. The inner channels of the shaft 14 can also contain components needed to operate the optical image gathering unit 18, as described below. One skilled in the art will appreciate that the shaft 14 can be made from a variety of sterilizable, biocompatible materials that have properties sufficient to enable the shaft 14 to be inserted and moved within tortuous channels of a body lumen. In one embodiment, the shaft 14 can be made from a polymer that has a low coefficient of friction. Alternatively, the shaft 14 can be made of a material that is coated with a low friction material, such as polytetrafluoroethylene.

The tip 16 can be formed as a unit with the shaft 14, or it can be separately attached. In one embodiment, the tip 16 includes a series of slots 39 that facilitate bending and flexing of the tip 16. One skilled in the art will appreciate that the tip 16 can be made from the same or different materials that are used to form the shaft 14.

The optical image gathering unit 18 can have a variety of configurations, shapes, and sizes, and it can be attached to the distal end of the tip 16 using a variety of techniques. By way of non-limiting example, the optical image gathering unit 18 can be mated onto the distal end of the tip 16 using male and female connectors that enable temporary or permanent attachment to the tip 16. Alternatively, the optical image gathering unit 18 can be attached in a variety of other ways, such as adhesive bonding, that enable the optical image gathering unit 18 to be mounted to the tip 16. Although generally described herein as being attached to a distal surface of the tip 16, one skilled in the art will appreciate that the optical image gathering unit 18 can be adapted to other, generally distal, portions of the tip 16. Although FIGS. 1, 4A, and 4B illustrate the optical image gathering unit 18 as a generally cylindrical structure that fits onto a distal end of the tip 16, the unit 18 can have a variety of other shapes and sizes. For example, the unit 18 can be sized and/or shaped to occupy less than the entire distal-facing surface of the tip 16.

The optical image gathering unit 18 can have a number of features and capabilities to facilitate acquisition of images during an endoscopic procedure. In one embodiment, shown in FIG. 4A, the optical image gathering unit 18 includes a lens 42 disposed on the distal end surface of the optical image gathering unit 18 to acquire and focus the images collected by the unit 18. The optical image gathering unit 18 can include zooming capability, for example, with a piezoelectric material disposed under the lens 42 that is adapted to move the lens 42 to facilitate zoom. A lens cleaning sprayer 44 can be disposed on the distal end surface of the unit 18 in close proximity to the lens 42 such that it is able to clean the lens 42 during an endoscopic procedure. In one exemplary embodiment, the optical image gathering unit 18 can also include an LED 46, shown in FIG. 4B, disposed on the distal end of the unit 18. The LED 46 is adapted to illuminate the area surrounding the unit 18 during an endoscopic procedure, allowing for the acquisition of improved images from the area.

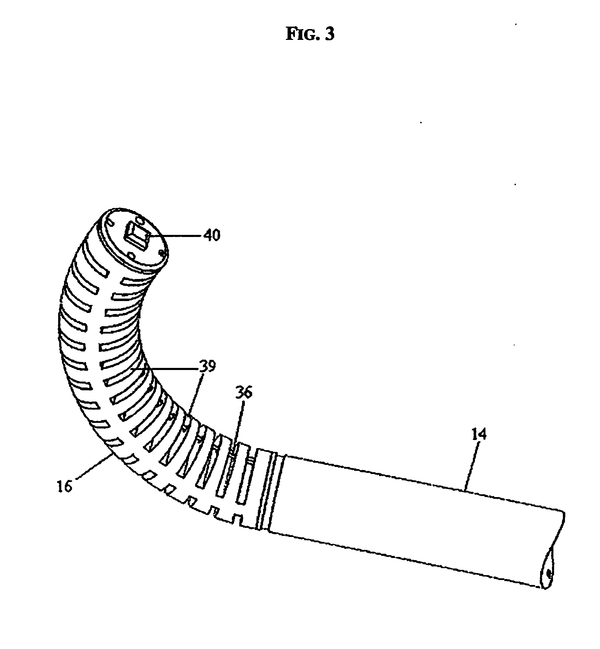

As previously indicated, the optical image gathering unit 18 can be disposed on a portion of the tip 16, as exemplified in FIGS. 1 and 3, which is selectively bendable and flexible to allow for the reorientation of the optics unit 18 for capturing images in an array of locations. The flexing and bending of the tip 16 can be controlled by a control member disposed on the handle 12, such as a thumb wheel 26 shown in FIGS. 1-2. A person skilled in the art will appreciate that the control member can have any configuration or shape that is capable of controlling the flexing and bending of the tip 16. In one embodiment, one or more cables 36 can extend from the flexible tip 16 to the thumb wheel 26. The thumb wheel 26 can rotate about an axle 28 disposed through the thumb wheel 26, causing tensioning and/or relaxing of the cables 36 to flex the tip 16. In one embodiment, two cables are positioned at opposed sides of the tip 16. To bend the tip 16 in one direction, the cable on that side of the tip 16 is tensioned while the other cable is slackened. One skilled in the art will appreciate that a number of other cables can be utilized as well, to enable bending of the tip 16 in multiples planes.

A CCD chip 40 for digital imaging, shown in FIG. 3, can be disposed on the endoscopic device 10, such as on a distal end surface of the tip 16. A person skilled in the art will appreciate that the CCD chip 40 can be any CMOS or other type of chip that is capable of digital imaging. The CCD chip 40 receives as input the images acquired by the optical image gathering unit 18 and it is in electrical communication with a processor board (not shown) disposed in the endoscopic device 10, such as in the handle 12, which receives the digital images from the CCD chip 40 for processing and display on LCD display screen 20.

The LCD display screen 20 can be disposed on the proximal end of the handle 12 to display the acquired images to a user. A person skilled in the art will appreciate that the LCD display screen 20 can be disposed at any location on the handle 12 for viewing by the user, and that the LCD display screen can display any alternative information, including instructions from a manual describing the surgical steps to be performed during an endoscopic procedure. In one embodiment of the invention, the LCD display screen 20 is disposed on an LCD mount 22. The LCD mount 22 can be rotatably disposed on the proximal end of the handle 12 to allow the user to rotate the LCD display screen 20 for viewing as the endoscopic device 10 is moved and rotated during an endoscopic procedure. A person skilled in the art will appreciate that the LCD display screen 20 can be disposed anywhere on the handle 12, or it can be disposed on another portion of the device 10 or remotely located. For example, the LCD display screen can be a flip-out screen attached to a side portion of the handle 12. If the LCD display screen 20 is remotely located, it can receive information to be displayed from the device 10 by a variety of techniques, including wired or wireless communication. In addition to the LCD display screen 20, the acquired images can also be displayed on an additional remote screen by wired or wireless communication. By way of non-limiting example, the acquired images can be streamed, using, for example, a USB or NTSC format, from the processor board or other component disposed in the device to an external display screen so the acquired images can be viewed on both the LCD display screen 20 and the external display.

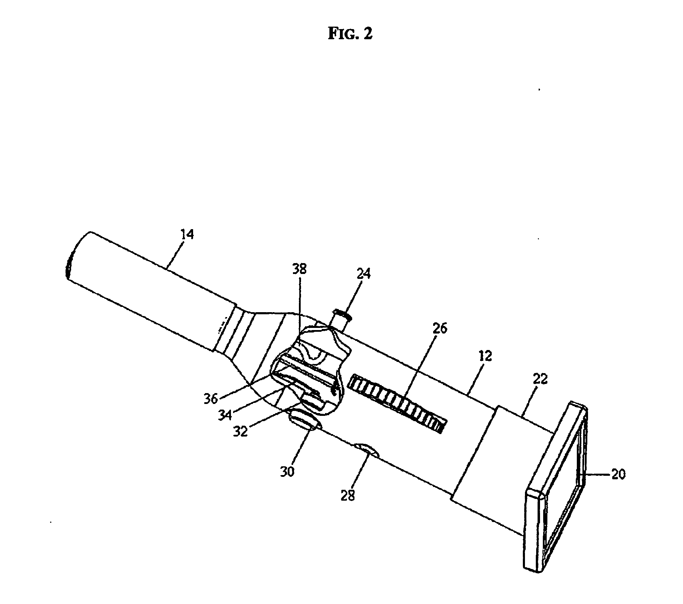

The lens cleaning sprayer 44 is in fluid communication with a fluid source coupled to the endoscopic device 10 through a fluid inlet port 24 disposed on the handle 12. Fluid for cleaning the lens 42 is delivered from the fluid inlet port 24 through a fluid delivery tube 38 (shown in FIG. 2) to the lens cleaning sprayer 44. A person skilled in the art will appreciate that the lens cleaning sprayer 44 can have any configuration or shape that is capable of delivering the cleaning fluid to the lens 42, and that a variety of controls, on or remote from the endoscopic device 10, can be used to selectively activate the sprayer 44.

The LED 46 for illuminating the area surrounding the optics unit 16 can be controlled by an actuator 30 disposed on the handle 12. The actuator 30 controls an energy source for delivering energy to the LED 46. The energy source can be provided from a variety of sources, such as from an outlet or an internal or external battery source. For example, the energy source can be supplied from a battery 32 disposed in the handle 12 as shown in FIG. 2. The battery 32 is in communication with the LED 46 though one or more wire leads 34 which extend from the LED 46 to the battery 32 through the shaft 14. By way of non-limiting example, the actuator 30 can be in the form of a button, a switch, a knob, or any other configuration to allow for the control of energy from the energy source to the LED 46. In lieu of an actuator disposed on the handle 12, one skilled in the art will appreciate that the actuator 30 can be located elsewhere, including on a foot pedal.

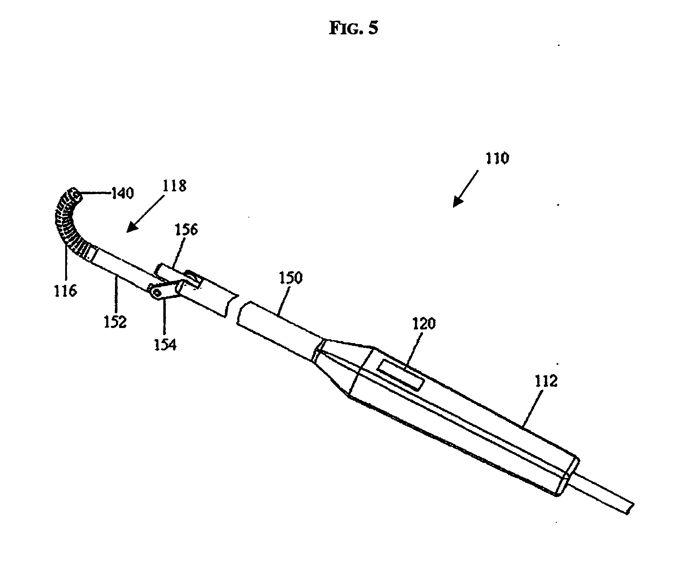

FIGS. 5-7 illustrate another embodiment of an endoscopic system which includes a disposable optics module. The endoscopic device 110 includes a handle 112 adapted to allow a user to grip the endoscopic device 110, and a flexible insertion shaft 150 extending from the handle 112. The shaft 150 includes at least one inner lumen (not shown) through which a scope 156 or other instruments, liquids, and/or gases can be passed during an endoscopic procedure. The endoscopic device 110 also includes a separately attachable optics module 118 that can be attached to the shaft 150 by an articulating link 154. The optics module 118 includes at its distal end a flexible tip 116 which is integral with or attached to a proximally positioned flexible shaft 152. As shown in FIG. 5, the articulating link 154 is pivotally coupled on its proximal end to the distal end of the shaft 150 and on its distal end to the proximal end of the shaft 152. As such, the optics module 118 is offset from the scope 156 or other instrument passed through the shaft 150.

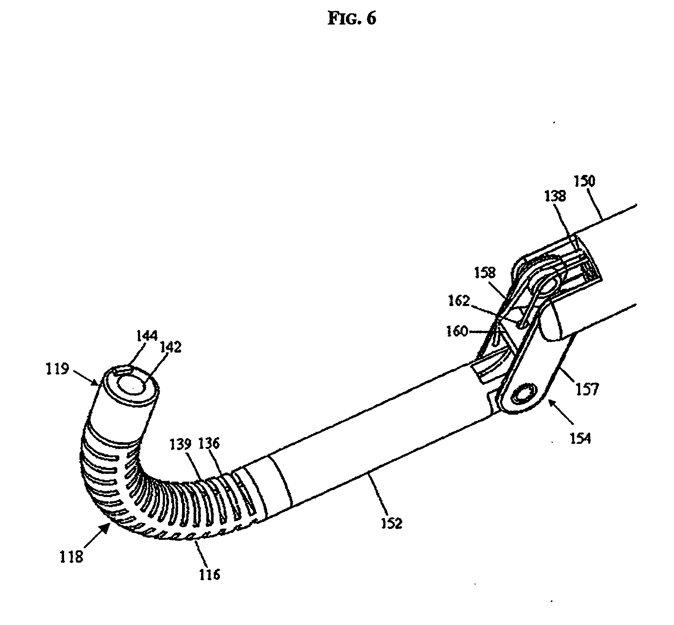

As shown in FIG. 6, the optics module 118 includes at its distal end an image gathering unit 119, which is in communication with a display screen. The display screen can be an LCD display screen 120 disposed on the handle 112, or it can be disposed on another portion of the device 110 or remotely located.

The flexible shaft 152 and the tip 116 can be similar in construction and function to the shaft 14 and tip 16 described above with respect to FIGS. 1-4. That is, the shaft 152 and the tip 116 can bend and be flexed to enable navigation of the device through a tortuous body lumen. In addition, the tip 116 can be selectively flexed and reoriented by a surgeon to position the image gathering unit 119 to capture desired images.

The articulating link 154 can include first and second links 157, 158 to couple to opposing sides of the shaft 150 and the shaft 152. The first and second links 157, 158 each include a distal pivot point and a proximal pivot point for pivotally coupling to the shaft 150 and to the endoscopic shaft 152. The articulating link 154 can also include a securing portion 160 disposed between the first and second links 157, 158, which can include a bore 162 for securing components that extend from the tip 116 and the optics module 118 to the handle 112.

The image gathering unit 119 can be a disposable component that is mated to the distal end of the tip 116. It can have a variety of configurations, shapes, and sizes, and it can be attached to the distal end of the tip 116 using a variety of techniques. By way of non-limiting example, the image gathering unit 119 can be mated onto the distal end of the tip 116 using male and female connectors that enable temporary or permanent attachment to the tip 116. Alternatively, the image gathering unit 119 can be attached in a variety of other ways, such as adhesive bonding, that would allow the image gathering unit 119 to attached to the tip 116. A disposable image gathering unit 119 can be advantageous due to the difficulty of re-sterilizing optics following an endoscopic procedure. A person skilled in the art will appreciate that the image gathering unit 119 can be disposable, or the entire optics module 118 can be disposable, which can include the image gathering unit 119, the tip 116, and the shaft 152.

The image gathering unit 119 can include a number of features to facilitate the acquisition of images during an endoscopic procedure. In one embodiment, the image gathering unit 119 can include features similar to the optical image gathering unit 18 described above with respect to FIGS. 1-4. For example, the image gathering unit 119 can include a lens 142 and a lens cleaning sprayer 144 shown in FIG. 6, which are similar to the lens 42 and sprayer 44 described above with respect to FIGS. 4A-4B. The image gathering unit 119 can also include zooming capability, for example, with a piezoelectric material disposed under the lens 142 that is adapted to move the lens to facilitate zoom. The image gathering unit 119 can optionally include an LED as similarly described above with respect to LED 46 in FIG. 4B. In the embodiment of the invention shown in FIGS. 5-7, the image gathering unit 119 can also include a CCD chip 140 for digital imaging, resulting in a disposable image gathering unit 119 that includes the optical components and the camera component. A person skilled in the art will appreciate that the CCD chip 140 can be any CMOS or other type of chip that is capable of digital imaging, and that the CCD chip 140 can be disposed on a variety of locations on the optics module 118, for example, on the distal end of the tip 116.

Similar to the embodiment described above, fluid is delivered to the lens cleaning sprayer 144 through a fluid delivery tube (not shown) extending from the lens cleaning sprayer 114 through the flexible tip 116, the shaft 152, the articulating link 154, and the shaft 150 to a fluid inlet port (not shown) disposed on the handle 112, which is coupled to a fluid source to provide cleaning fluid to the lens cleaning sprayer 144. A person skilled in the art will appreciate that the lens cleaning sprayer 144 can have any configuration or shape that is capable of delivering the cleaning fluid to the lens 142, and that a variety of controls, on or remote from the endoscopic device 110, can be used to selectively activate the sprayer 144. The LED can receive energy from wire leads (not shown) extending from the LED through the tip 116, the shaft 152, the articulating link 154, and the shaft 150 to an energy source (not shown), such as the battery described above, disposed in the handle 112. A person skilled in the art will appreciate that the energy source can be provided from a variety of sources, such as from an outlet or an internal or external battery source. An actuator (not shown) can be disposed on the handle 112 to control the energy source for delivering energy to the LED. By way of non-limiting example, the actuator can be in the form of a button, a switch, a knob, or any other configuration to allow for the control of energy from the energy source to the LED. In lieu of an actuator disposed on the handle 12, one skilled in the art will appreciate that the actuator 30 can be located elsewhere, including on a foot pedal.

The CCD chip 140 is in electrical communication with a processor board (not shown) disposed in the handle 112, which receives the digital images from the CCD chip 140 for processing to be displayed on the LCD display screen 120. A person skilled in the art will appreciate that the LCD display screen 120 can be disposed anywhere on the handle 112, or it can be disposed on another portion of the device 110 or remotely located. For example, the LCD display screen can be a flip-out screen attached to a portion of the handle 112. If the LCD display screen 120 is remotely located, it can receive information to be displayed from the device 110 by a variety of techniques, including wired or wireless communication. In addition to the LCD display screen 120, the acquired images can also be displayed on an additional remote screen by wired or wireless communication. A person skilled in the art will appreciate that the LCD display screen 120 can display images or information from various sources. For example, the LCD display screen 120 can display images acquired by the image gathering unit 119, or the scope 156. While FIG. 5 illustrates the scope 156 in an inner lumen of the shaft 150, the scope 156 can be removed or absent altogether and other instruments can be passed through the inner lumen and their use can be imaged by the image gathering unit 119. In addition to the LCD display screen 120, the acquired images can also be displayed on an additional remote screen by wired or wireless communication. By way of non-limiting example, the acquired images can be streamed, using, for example, a USB or NTSC format, from the processor board or other component disposed in the device to an external display screen so the acquired images can be viewed on both the LCD display screen 120 and the external display.

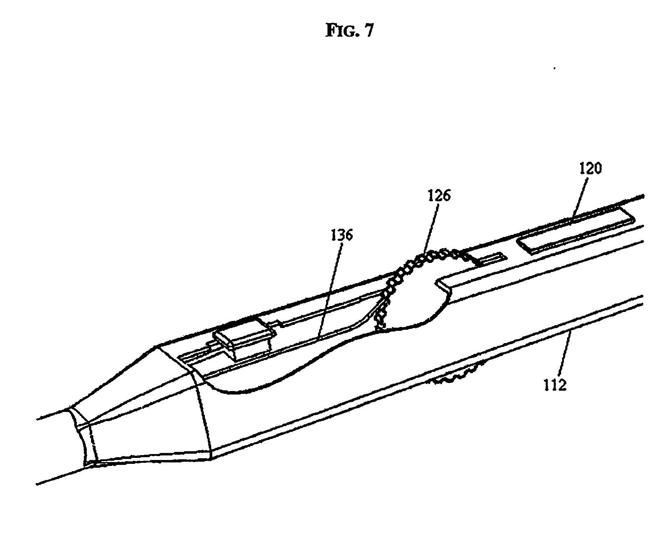

The image gathering unit 119 is disposed on the distal end of the flexible tip 116, shown in FIGS. 5-6, which extends from the distal end of the secondary shaft 152. The flexible tip 116 is flexible to allow for the reorientation of the image gathering unit 119 as similarly described above in FIGS. 1 and 3. The bending and flexing of the tip 116 is controlled by a control member disposed on the handle 112, such as the thumb wheel 126 shown in FIG. 7, similar to the tip 16 and its controls as described above. A person skilled in the art will appreciate that the control member can have any configuration or shape that is capable of controlling the bending and flexing of the tip 16. Similar to the description above, cables 136 can extend from the thumb wheel 126 to the flexible tip 116, which includes a series of adjacent slots 139 which allow the flexible tip 116 to flex when the cables 136 are pulled by movement of the thumb wheel 126. In this embodiment shown in FIGS. 6-7, the cables extend from the flexible tip 116 through the secondary shaft 152, the articulating link 154, and the shaft 150 to the thumb wheel 26. The cables 136 are secured by passing through the bore 162 of the articulating link 154 to prevent the cables 136 from slipping as they are pulled by the movement of the thumb wheel 126.

The endoscopic devices 10, 110 shown in FIGS. 1 and 5 are intended to be used to access tubular organs through an existing natural orifice. However, the devices 10, 110 can also be adapted for use in other procedures, such as laparoscopic procedures, where a surgical opening is created in a patient to access a part of the body. As such, the devices 10, 110 can be sized and shaped to be optimized for their intended use. For example, for trans-oral and trans-anal devices, the shafts 14, 150 should be thin, flexible, and elongate to allow access to various parts of a patient's body to perform a variety of procedures.

As noted above, a feature of the endoscopic devices described herein is that they are entirely or at least partially disposable. Nevertheless, it is also envisioned that the endoscopic devices described herein, including portions thereof, can be designed to be disposed after a single use, or can be designed to be used multiple times. In either case, however, the device can be reconditioned for reuse after at least one use. Reconditioning can include any combination of the steps of disassembly of the device, followed by cleaning or replacement of particular pieces, and subsequent reassembly. By way of example, the endoscopic devices can be reconditioned after the device has been used in a medical procedure. The device can be disassembled, and any number of the particular pieces (e.g., the optics unit 18 and the optics module 118) can be selectively replaced or removed in any combination. For example, the optics can be replaced. Upon cleaning and/or replacement of particular parts, the device can be reassembled for subsequent use either at a reconditioning facility, or by a surgical team immediately prior to a surgical procedure. Those skilled in the art will appreciate that reconditioning of an endoscopic device can utilize a variety of techniques for disassembly, cleaning/replacement, and reassembly. Use of such techniques, and the resulting reconditioned endoscopic device, are all within the scope of the present application.

Although the invention has been described in the context of a medical treatment device being an endoscope, the optical capabilities can be included with any diagnostic or treatment device used in minimally invasive surgical techniques, such as endoscopic and laparoscopic techniques. By way of non-limiting example, such diagnostic or treatment devices can include suture delivery devices and delivery devices for clips, staples, and other surgical fasteners.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A medical treatment device, comprising:
an elongate flexible insertion element adapted for delivery within a patient;
an optical image gathering unit disposed on a distal portion of the insertion element, the optics unit adapted to acquire images during medical treatment procedures; and
an image display screen disposed on a proximal portion of the medical treatment device adapted to communicate with the optical image gathering unit to display the acquired images.

2. The device of claim 1, wherein the optical image gathering unit includes a lens disposed on a distal end thereof adapted to focus images and an LED disposed on the distal end thereof adapted to illuminate the area surrounding the optical image gathering unit.

3. The device of claim 2, wherein the optical image gathering unit further includes a lens cleaning sprayer disposed on the distal end thereof adapted to clean the lens.

4. The device of claim 3, wherein the lens cleaning sprayer is coupled to a fluid inlet port disposed on the proximal end of the device through a fluid delivery device adapted to deliver lens cleansing fluid to the lens cleanser.

5. The device of claim 1, wherein the optical image gathering unit is disposed on at least a portion of a flexible tip located on the distal end of the insertion element.

6. The device of claim 5, wherein the insertion element includes one or more cables disposed therein and attached to a control member disposed on the proximal portion of the device, the cables being adapted to effect bending of a distal portion of the insertion element in response to actuation of the control member.

7. The device of claim 5, wherein a digital imaging chip is disposed on the distal portion of the flexible tip to collect images from the lens.

8. The device of claim 7, wherein the digital imaging chip is in electrical communication with the image display screen to allow the image display screen to display images acquired by the optical image gathering unit.

9. The device of claim 8, wherein the digital imaging chip communicates with the image display screen using wireless RF technology.

10. The device of claim 8, wherein the digital imaging chip is in electrical communication with a processing board disposed in the proximal portion of the device through one or more wires adapted to process the images and transmit the images to the image display screen.

11. An optics module for use with a medical treatment device, comprising:
a lens disposed on a distal end of an optics unit adapted to acquire images, and
wherein the optics module is disposable and is adapted to be removably and replaceably mated to a distal end of a medical treatment device such that it is able to communicate with an image display screen to display images acquired by the optics module.
